# EUROPEAN PATENT SPECIFICATION

(11) **EP 1 651 266 B1**
(45) Date of publication and mention of the grant of the patent: **03.03.2010**
(21) Application number: 03740913.3
(22) Date of filing: 25.07.2003
(51) Int. Cl.: A61K 39/395, A61P 37/06, C07K 16/24

(54) **ADMINISTRATION OF ANTI-TNF-ALPHA F(AB')2 ANTIBODY FRAGMENTS**
VERABREICHUNG VON ANTI-TNF-ALPHA F(AB')2 ANTIKÖRPER-FRAGMENTEN
ADMINISTRATION DE FRAGMENTS D'ANTICORPS F(AB')2 ANTI-TNF-ALPHA

(43) Date of publication of application: 03.05.2006
(73) Proprietor: Laboratorios Silanes, S.A. de C.V., Mexico, D.F., C.P. 03100 (MX)
(72) Inventor: Juan Lopez de Silanas, Mexico D.F. 06600 (MX); Jorge F. Paniagua Solis, Delegacion Tlalpan (MX); Alberto Diaz-Quinonez, Delegacion Gustavo A. Madero (MX); Rita Mancilla Nava, Delegacion Tlalpan (MX)
(74) Representative: Grünecker, Kinkeldey, Stockmair & Schwanhäusser Anwaltssozietät
(86) International application number: PCT/IB2003/002971
(87) International publication number: WO 2005/009464

(56) References cited:
- WO-A-98/22137
- WO-A1-92/22324
- WO-A1-02/096461
- WO-A1-02/098460
- US-A1- 2002 077 276
- US-A1- 2002 136 723
- US-B1- 6 448 054
- MISISCHIA RICHARD J ET AL: "Rheumatoid arthritis: developing pharmacological therapies." EXPERT OPINION ON INVESTIGATIONAL DRUGS. JUL 2002, vol. 11, no. 7, July 2002 (2002-07), pages 927-935, XP002385077 ISSN: 1354-3784
- MAINI RAVINDER N ET AL: "How does infliximab work in rheumatoid arthritis?" ARTHRITIS RESEARCH. 2002, vol. 4 Suppl 2, 2002, pages S22-S28, XP002385078 ISSN: 1465-9905
- MOURA-DA-SILVA A M ET AL: "Processing of pro-tumor necrosis factor-alpha by venom metalloproteinases: a hypothesis explaining local tissue damage following snake bite." EUROPEAN JOURNAL OF IMMUNOLOGY. SEP 1996, vol. 26, no. 9, September 1996 (1996-09), pages 2000-2005, XP008065161 ISSN: 0014-2980
- SKURKOVICH S. ET AL: 'Treatment of Corneal Transplant Rejection in Humans With Anti-interferon-GAMMA Antibodies' AMERICAN JOURNAL OF OPHTAMOLOGY vol. 133, no. 6, June 2002, pages 829 - 830
- YU C.-K. ET AL: 'DERMATOPHAGOIDES-FARINAE-INDUCED PULMONARY EOSINOPHILIC INFLAMMATION IN MICE' INTERNATIONAL ARCHIVES OF ALLERGY AND IMMUNOLOGY vol. 112, no. 1, 1997, pages 73 - 82, XP000953027
- QIAN Y. ET AL: 'Topical soluble tumor necrosis factor receptor type I suppresses ocular chemokine gene expression and rejection of allogeneic corneal transplants' ARCHIVES OF OPHTHALMOLOGY vol. 118, no. 12, 2000, pages 1666 - 1671, XP008085330
- PRAUSNITZ M.R.: 'Reversible Skin Permeabilization for Transdermal Delivery of Macromolecules' CRITICAL REVIEWS IN THERAPEUTIC DRUG CARRIER SYSTEMS vol. 14, no. 4, 1997, pages 455 - 483, XP008008703
- SCALLON B. ET AL: 'Binding and functional comparisons of two types of tumor necrosis factor antagonists' JOURNAL OF PHARMACOLOGY AND EXPERIMENTAL THERAPEUTICS vol. 301, no. 2, 01 May 2002, pages 418 - 426, XP002307181

## Description

### BACKGROUND OF THE INVENTION

### Field of the Invention

The present invention is directed to the use of anti-TNF-α F(ab')₂ neutralizing antibody fragments in the preparation of a medicament for topical administration for treating an TNF-α- mediated immune reaction which is due to a rejection of a corneal transplant. The antibody fragments are preferably substantially free from albumin and of whole antibodies, as well as substantially free of pyrogens, and may be administered with an effective amount of a pharmaceutically acceptable carrier.

### Background Art

Cytokines play a key role in many biological processes such as the induction of the immune response, recruitment of inflammatory cells, cytotoxicity, anti-viral activity, wound repair, angiogenesis, apoptosis, fever, and synthesis of acute-phase proteins. Cytokines act in a complex network in which they can induce the production and secretion of other cytokines, modulate the expression of cytokine receptors, and are capable of having synergistic or antagonizing effects on other cytokines. Proinflammatory cytokines have also been implicated as mediators of transplant rejection.

Antibodies are proteins of a globulin type known as immunoglobulins (Igs) that are present in blood serum as a response of the immune system to the invasion of some foreign substance or organism. They are characterized for specific combination with substances that are foreign to the organism, to neutralize and precipitate them with the purpose of removing them from circulation. Various industrial applications have been developed utilizing antibodies, such as diagnosis, monitoring, prevention and treatment of a variety of ailments.

In regions where, due to climatic conditions, venomous animals abound, antibodies have been given a special use to combat venom in treatment of patients with scorpion, spider and snake stings or bites. Another use that is developing is treatment of autoimmune diseases such as rheumatoid arthritis, immune-dependent diabetes mellitus, AIDS, hemophilic anemias, rheumatic fever, multiples sclerosis, thyroiditis and psoriasis, among others. In these cases, anti-cytokine antibodies are applied either directly to the patient or by extracorporeal immunosorption. Ideally, such treatment will remove the cytokines generated by the organism in response to an ailment to avoid death or an acute immune reaction (*see* U.S. Pat. No. 5,888,511 and 4,940,670).

The concept of anticytokine therapy to combat human disease originated with the use of steroids in canine and primate models of sepsis. Sepsis, also known as systemic inflammatory response syndrome (SIRS) is the systemic host response to an overwhelming infection of the bloodstream by toxin-producing bacteria. Severe sepsis can develop into septic shock, which occurs when an overwhelming infection leads to low blood pressure, low blood flow and organ dysfunction.

Septic shock causes more than 100,000 deaths per year in Latin America. The pathogenesis of sepsis typically includes exposure to a noxious element, classically an endotoxin, followed by an inflammatory and anti-inflammatory immune response by the host. The mechanisms associated with injury during the septic process are triggered by bacterial antigens such as lipopolysaccarides (LPS), which induce the overproduction of proinflammatory cytokines such as TNF-α. Severe septic shock may result in the failure of such vital organs as the brain, heart, kidneys and liver. Although the complete neutralization of TNF-α will often give rise to bacterial infections, low doses of anti-cytokine antibodies may attenuate the early proinflammatory response, which is beneficial for preventing both sepsis and septic shock.

Infusion of endotoxin (such as venom) in both animals and man elicits a measurable release of cytokines such as tumor necrosis factor-alpha (TNF-α) and interleukin-1 beta (IL-1β). Blood levels of TNF-α in septic patients have been correlated with both severity of disease and mortality. TNF-α infusion reproduces the tachycardia, hypotension, leukocytosis, coagulopathy, increased permeability, pulmonary edema, and multiple organ failure associated with sepsis. Anti-TNF-α therapy in the form of whole antibodies specific to the TNF-α has protected animals from death due to endotoxin infusion. Interleukin-1β also induces all the signs and symptoms of sepsis, such as fever, anorexia, and hypotension. Blockade of IL-1β using a soluble IL-1 receptor antagonist (IL-1Ra) has attenuated the severity of disease and mortality in experimental animal models of shock and sepsis. Thus, neutralization or removal of cytokines such as IL-β1 and TNF-α may impart resistance to endotoxic shock.

Conventional treatment for snake bites and scorpion stings primarily includes the use of anti-venoms, steroids and antibiotics. These treatments usually must occur within two hours of the bite. Possible complications to insect bites and stings include allergic reaction, infection, disease, and reaction to the venom such as toxic reaction or shock. Local tissue effects vary among scorpion (and spider or snake) species. Other effects may include hypertension or hypotension, respiratory distress or the loss of protective airway reflexes, pulmonary edema and various autonomic effects. Many of these complications may be avoided or minimized by immediate neutralization of cytokines.

In the past, "anti-venoms" to snake bites have been obtained by hyperimmunizing horses with snake venom and then given to individuals bitten by poisonous snakes. Although the antibodies in the horse serum neutralized the toxins in the snake venom, other horse antibodies sometimes remained in the individual for as long as several weeks. A condition called "serum sickness" sometimes resulted in response to the injection of serum (which includes whole antibodies) from a foreign species into a patient in need of treatment. The differences in the primary amino acid sequence of horse immunoglobulin and human immunoglobulin caused the individual to begin to make antibodies against the foreign horse immunglobulin and other serum proteins present in the injection. Serum sickness would begin to manifest itself 7 -10 days after the initial injection. At this time, the individual would begin to show symptoms such as fever, chills, and sometimes glomerularnephritis. Although symptoms would often resolve themselves after removal of the foreign antigen from the individual's blood, re-exposure to the same antigen could result in accelerated and potentially more severe symptoms (*i*.*e*. kinetics similar to a secondary immune response). More recently, antibody generation has been achieved using recombinant DNA constructions that express peptides of the venoms. This technology has been most extensively used against the group of inflammatory cytokines that includes TNF-α, IFN-γ, IL-1 and IL-6.

Psoriasis vulgaris is T cell-mediated inflammatory disease in humans. The pathogenesis of psoriasis is linked to activation of several types ofleukocytes that control cellular immunity and to a T cell-dependent inflammatory process in skin that accelerates the growth of the epidermal and vascular cells in psoriasis lesions. Critical steps in immunologic activation include Langerhans cell maturation, T cell activation, differentiation and expansion of Type 1 T cells, selective trafficking of activated T cells to skin, and induction of inflammatory cytokine and chemokine cascade in skin lesions. Both IFN-γ and TNF-α are expressed at increased levels in psoriatic lesions, and expression of several genes regulated by nuclear factor κ β (transduces TNF-α signals) is also increased. Because the cytokines secreted by activated T cells drive inflammatory responses and end-stage immune responses, one therapeutic strategy is to selectively deactivate specific cytokines with whole antibodies. Once again, treatment with whole antibodies increases the risk of a separate immune reaction to the foreign antibodies themselves.

Proinflammatory cytokines also play an important role in the biological processes involved in corneal immune and inflammatory responses, which are often associated with corneal transplant. Corneal cells have been reported to be able to synthesize a variety of cytokines and growth factors, including IL-1, IL-6 and TNF-α.

When a tissue or organ, such as a patch of skin or a cornea, is grafted from one animal to another, the organ may be accepted by the recipient without any immune reaction. Alternatively, the graft may be rejected by the recipient at varying rates which depend on the underlying effector mechanisms. Hyperacute rejection occurs very rapidly in patients who already have antibodies against a graft, and may be avoided by ABO matching and by performing cross-matching, in which serum from a prospective recipient is tested for the presence of cytotoxic anti-donor antibodies. An acute reaction takes days or weeks to manifest and is due to the primary activation of T cells, which trigger various effector mechanisms including cytokine production. Alpha tumor necrosis factor (TNF-α) plays an active role in acute graft rejection, which indicates that neutralization or minimization of TNF-α might help to prevent such acute graft rejections. Interleukin-1α has also been implicated in the early alloimmune response to corneal grafts, thus neutralization or minimization of IL-1α may also aid in the prevention of corneal graft rejections. (*See* Zhu, et al., J. Ifn. & Cytokine Rsrch., 19:661-669 (1999)). Chronic rejection is primarily attributed to genetic disparities between the graft donor and recipient, and most commonly treated with immunosuppressive drugs.

Treatment of autoimmune diseases, including AIDs, by blocking, inhibiting, neutralizing or removing harmful interferons, tumor necrosis factors and other pathological immunogens or factors has been investigated, but has not found widespread success due to complications often associated with immunotherapy. *(See* U.S. Patent No. 5,888,511). One method of treatment described comprises administering to a patient an effective amount of anti-IFN-γ antibodies and/or receptor to treat an IFN-γ mediated autoimmune disease. Such treatment with whole antibodies or receptors may cause its own immune reaction if the patient recognizes the antibodies as foreign, which would be counterproductive in the overall treatment strategy for the underlying autoimmune disease.

Rhuematoid Arthritis (RA) is a common, frequently severe, chronic inflammatory disease. In RA, cytokines are involved in almost all aspects of synovial inflammation and destruction of articular tissues, with tumor necrosis factor (TNF) playing a particularly important role. Neutralizing TNF in patients with RA by means of intravenous infusion of soluble TNF receptors or anti-TNF antibodies has shown some promise, but often has induced a separate immune response. (*See* Fox, D., Arch Intern. Med. 160: 437-444 (2000)). Thus, although such treatments are theoretically capable of neutralizing some of the cytokines involved in RA, the possibility of causing a separate immune reaction has prevented widespread success of such treatments. Administration of a composition comprising F(ab')₂ antibody fragments allows for the effective neutralization of those cytokines associated with RA, while substantially decreasing the possibility of a separate immune reaction as described above.

Sepsis has also evaded a consistently successful therapy approach despite clinical trial efforts spanning back over twenty years. Steroid agents such as corticosteroids have been tested in many stages of sepsis, but have failed to demonstrate any significant treatment benefits. *(See* Martin, Greg S., CHEST, Current Management Strategies for Severe Sepsis and Septic Shock (2001)). Antibiotic therapy combined with source control and removal of infections (when possible) are traditional treatments that have met with some minimal success when applied in the very early stages of sepsis. (*Id*.). Immunotherapy approaches ranging from glucocorticoid administration to monoclonal antibody preparations have also been associated with such adverse effects as compounding the immune reaction, and thus have not found widespread utility. (*See* Zeni et al., Crit. Care Med. 25:1095-1100 (1997)).

Skurkovich S, Kasparov A, Narbut N, Skurkovich B: "Treatment of Corneal Transplant Rejection in Humans with Anti-interferon-GAMMA Antibodies" America Journal of Ophthalmology, vol. 133, no. 6, June 2002 (2002-06), pages 829-830, discloses the treatment of a corneal transplant rejection in humans by administering anti-interferon-gamma F(ab')₂ antibodies.

Qian Y: Dekaris I: Yamagami S; Dana M R: "Topical Soluble tumor necrosis factor receptor type I suppresses ocular chemokine gene expression and rejection of allogeneic corneal transplants" Archives of Ophthalmology, vol. 118, no. 12, 2000, pages 1666-1671 describes topical treatment with soluble TNF receptor type 1 of allogeneic corneal transplants.

### SUMMARY OF THE INVENTION

Treatment with anti-cytokine F(ab')₂ antibody fragments allows for the neutralization of excess cytokines while minimizing the risk of a separate immune reaction to the fragments themselves. In one aspect, the invention relates to the use of anti-TNF-α F(ab')₂ neutralizing antibody fragments in the preparation of a medicament for topical administration for treating an TNF-α- mediated immune reaction which is due to a rejection of a corneal transplant, wherein the medicament is prepared for direct application to the eye.

### DETAILED DESCRIPTION OF THE PREFERRED EMBODIMENTS

The following glossary is provided as an aid to understand certain terms used herein. The explanations provided in the glossary are for illustrative purposes and do not limit the scope of the invention.

The terms "patient" and "individual" are interchangeably used to mean a warm-blooded animal, such as a mammal, suffering from a bite or sting from a venomous animal, or who is suffering from a disease such as an autoimmune disease or "graft versus host" disease, or is in danger of rejection of a transplanted allogeneic tissue or organ. It is understood that humans and animals are included within the scope of the terms "patient" or "individual," unless otherwise specified.

An "immunogen" is any cell or molecule that will provoke an immune response (production of antibodies and/or sensitization of lymphoid cells) in an immunologically competent individual.

A "cytokine-mediated immune reaction" is the immunological response and the following inflammatory processes that are orchestrated by the group of low molecular weight proteins known as cytokines. This could include a reaction caused by the bite of a venomous animal, sepsis, septic shock, as well as a variety of autoimmune diseases such as rheumatoid arthritis or psoriasis. Such reactions may also include acute rejection of a tissue, graft or prosthesis transplantation.

"Anaphylactic shock" is a severe generalized form of anaphylaxis in which there is widespread release of histamine and other vasoactive substances causing edema, constriction of the bronchioles, heart failure, circulatory collapse, and sometimes death.

"Anaphylaxis" is extreme immunological sensitivity of the body or tissues to reintroduction of an antigen. It is a form of anamnestic reaction and is accompanied by pathological changes in tissues or organs due to the release of pharmacologically active substances such as histamines and/or cytokines.

"Cytokines" are any of a varied group of hormone-like proteins that are produced and secreted by mammalian cells and have autocrine or paracrine activity. They elicit from the target cell a variety of responses depending on the cytokine and the target cell. For example, cytokines play a role in the immune response to an infection or infectious organism/agent. Cytokines act in a complex network in which they can induce the production and secretion of other cytokines, modulate the expression of cytokine receptors, and are capable of having synergistic or antagonistic effects on other cytokines. Thus, cytokines help to regulate the intensity and duration of an immune response.

An "antigen" is a molecule which reacts with preformed antibody or antibody fragment (such as F(ab')₂) and the specific receptors on T and B cells.

An "immune response" in vertebrate animals can be defined as the acquired, transferable capacity of lymphocytes to react with specificity and memory to foreign substances.

"Lymphocytes" are a class of leukocytes (white blood cells) that recognize specific antigens by means of cell-surface receptors which they themselves have synthesized.

An "immunoglobulin" (Ig) is any member of a group ofproteins occurring in higher animals as major components of the immune system. They are produced by cells of the lymphocyte series, and virtually all possess specific antibody action. Each immunoglobulin molecule essentially comprises four polypeptide chains, two identical heavy chains and two identical light chains, linked together by disulfide bonds. In addition, all contain differing amounts of attached oligosaccaride. There are five classes of immunoglobulins, IgA, IgD, IgE, IgG, and IgM. The primary structures of the heavy chains differ among the various classes, being designated α, δ, ε, γ, and µ, respectively for the classes listed above. There are two types of light chains, κ and λ. IgA and IgM molecules contain multiples of the four-chain unit.

"Alpha Tumor Necrosis Factor or TNF-α" (formerly cachetin) is a cytokine that is produced by macrophages, monocytes, endothelial cells, neutrophils, smooth muscle cells, activated lymphocytes, and astrocytes. It is a transmembrane glycoprotein and cytotoxin with a variety of function, including the ability to mediate the expression of genes for growth factors, cytokines, transcription factors, and receptors. It can cause cytolysis of certain tumor cell lines, and has been implicated in the induction of cachexia. It is a potent pyrogen, causing fever by direct action or by stimulation of interleukin-1 secretion. It can also stimulate cell proliferation and induce cell differentiation under certain conditions. The molecule is a homotrimer.

An "interleukin" is any member of a heterogenous group of cytokines that have the ability to act as signaling molecules between different populations of leukocytes.

"Interleukin-1 or IL-1" (Former names include: lymphocyte-activating factor (LAF); mitogenic protein (MP); helper-peak-1 (HP-1); T-cell replacing factors II and m (TRF-III, TRF-M); and B-cell-activating/differentiation factor (BAF, BDF)). IL-1 is produced primarily by activated macrophages. It is not able to promote and maintain *in vitro* long-term cultures of T-cells, but stimulates thymocyte proliferation by inducing interleukin-2 release. It is involved in the inflammatory response, and is identified as an endogenous pyrogen.

"Interleukin-1-alpha or IL-1-α" is one of two molecular forms of interleukin-1.

"Interleukin-1-beta or IL-1-β" is the second of two molecular forms of interleukin-1.

"Interleukin-2 or IL-2"(Other names include: thymocyte-mitogenic factor (TMF); T-cell growth factor (TCGF); co-stimulator; killer-cell helper factor (KHF); secondary cytotoxic T-cell-inducing factor (SCIF)). Interleukin-2 is an interleukin that is produced by T cells in response to mitogenic or antigenic stimulation. IL-2 promotes and maintains *in vitro* long-term cultures of T-cells. The expression of both IL-2 and the IL-2 receptor by T cells is induced by IL-1.

"Interleukin-6 or IL-6" (Other names include B-cell stimulatory factor 2; interferon β-2; and hybridoma growth factor). IL-6 is an interleukin that induces myeloma and plasmacytoma growth, as well as nerve cell differentiation. In hepatocytes, it induces acute-phase reactants. IL-6 acts in synergy with IL-1 and TNF in many immune responses, including T cell activation. The main effect of IL-6 is to augment the responses of immune cells to other cytokines.

"Interleukin-12 or IL-12" (An alternative name is cytotoxic lymphocyte maturation factor). IL-12 is an interleukin that can act as a growth factor for activated T and natural killer cells (NK) cells. It can enhance the lytic activity of NK/lymphokine-activated killer cells, and stimulate the production of interferon-γ by resting PMBC. It is a disulfide-linked heterodimer of 40 kDa and 35 kDa subunits.

An "interferon or IFN" is any member of a group of proteins that form a closely related group of nonviral proteins that are produced and liberated by animal cells following exposure to a variety of inducing agents. They are not normally present in uninduced cells. Human cells produce three main types of interferon: interferon alpha (IFN-α), interferon beta (IFN-β), and interferon gamma (IFN-γ). Interferon alpha and beta are classified as Type I interferons, and interferon gamma is classified a Type II interferon.

A "Type II interferon" (also called inteferon gamma, or IFN-γ) is produced by lymphocytes activated by specific antigens or mitogens. In addition to antiviral activity it has important immunoregulatory functions, such as activation of macrophages. Interferon gamma is a glycoprotein that exists as a heterodimer. The term "substantially free" refers to the absence of pyrogen and protein material foreign to F(ab')₂ antibody fragments, such as albumin or whole antibodies, in accordance with Mexican pharmacopeia.

The term "aseptic conditions" refers to precautionary measures or methods employed in handing the different products from each step the contamination of culture or sterile media and infection by extraneous microorganisms or contaminants.

The term "effective amount" or "pharmaceutically effective amount" of a compound in unit dose of the composition depends upon a number of factors. Included among these factors are quantity of any other ingredients used and tolerance of the active ingredient of the composition. Effective amounts of the active ingredient ranges from about 0.1% to about 50% by weight based on the total weight of the composition. The amount of anti-cytokine F(ab')₂ preparation to be administered depends upon the species from which the venom was collected. For compositions against scorpions, the F(ab')₂ preparation to be filled in each flask is the amount necessary to neutralize from about 135 to about 220 lethal doses 50% (LD50s) of the venom. For compositions against the black widow spider, the amount necessary for neutralization is from about 540 to about 880 lethal doses 50% (LD50s) of the venom. For compositions against coral snakes, the amount necessary for neutralization is from about 360 to about 660 lethal doses 50% (LD50s) of the venom. For compositions against i-Bothrops and Crotalus, the flasks are filled with the amount necessary to neutralize from about 700 to about 1100 lethal doses 50% (LD50s) of the venom.

"Topical administration" includes the application of medicinal substances to the skin or various body orifices. Medications may be applied in a variety of forms such as liquid, semisolid or solid. Topical application of medications may deliver an active ingredient locally as well as systemically.

"Emollients" are bland, fatty or oleaginous substances which may be applied locally, particularly to the skin, and also to mucous membranes or abraded tissues. Water-soluble irritants, air and airborne bacteria are excluded by an emollient layer. Some examples of common emollients are castor oil, sulfated castor oil, cocoa butter, coconut oil, cold cream, corn oil, cottonseed oil, hydrophilic ointment, sesame oil, and theobroma oil.

A "pharmaceutically acceptable carrier" or "dermatologically acceptable carrier" is a solid or liquid filler, adjuvant, additive, excipient or substance which may be safely used in topical administration. A variety of pharmaceutically acceptable carriers that are well known in the art include solid or liquid fillers, diluents, emollients, hydrotropes, surface active agents, and encapsulating substances. The amount of carrier employed in conjunction with the F(ab')₂ fragments is an amount that is sufficient to provide a practical quantity of material per unit dose of composition.

Specific pharmaceutically acceptable carriers for topical administration that may be incorporated in the composition of the invention include solvents such as water, alcohols, alkyl methyl sulfoxides, pyrrolidones, laurocapram, and miscellaneous solvents such as acetone, dimethyl acetamide, dimethyl formamide

Some of the possible components of topical, transdermal and transmucosal formulations and delivery devices include solubilizing agents, suspending agents, dispersing agents, preservatives, animal and vegetable fats, oils, or waxes, stabilizing agents, thickening or gelling agents, buffering agents, adhesive agents, adjuvants and additives, emulsifiers and penetration enhancing agents. Some examples of conventional preservatives are methyl paraben, propyl and butyl imidazolidinylurea, methylchloroisothiazolinone and methylsiothiazolinone.

Examples of adjuvants and additives which may be added to topical delivery compositions include bactericides, fungicides, virucides, light filter substances, active ingredients with a cooling action, antioxidants, plant extracts, antiinflammatories, substances which promote wound healing, surface-active substances, emulfiers, emollients, moisturizers, humectants, fats, oils, waxes, alcohols, polyols, polymers, foam stabilizers, organic solvents, silicon derivatives or chelating agents.

Oils or waxes suitable for use in dermatological compositions include mineral oils (liquid petroleum), vegetable oils (liquid fraction of karite butter, sunflower oil), animal oils (perhydrosqualene), synthetic oils (purcellin oil), silicon oils or waxes and fluorinated oils.

Some emulsifiers which are suitable for use in dermatological compositions include glyceryl stearate, polysorbate 60, and sorbitan monostearate.

Exemplary solvents for use in topical compositions include the lower alcohols, such as ethanol, isopropanol and propylene glycol.

Hydrophilic gelling agents which may be used in topical compositions include carboxyvinyl polymers, acrylic copolymers, polyacrilamides, polysaccharides, natural gums and clays. Lipophilic gelling agents include modified clays, metal salts of fatty acids (such as ammonium stearates), and hydrophobic silica, ethylcellulose or polyethylene.

"Polyoxyalklyene block copolymers" are nonionic block copolymers of ethylene oxide and propylene oxide. The poly (oxyethylene) segment is hydorphilic and the poly (oxypropylene) segment is hydrophobic.

As described briefly above, there are five major classes of immunoglobulins, which are designated as IgM, IgG, IgA, IgE, and IgD. The heavy chains of Ig molecules within each class are designated γ (gamma), µ (mu), α (alpha), ε (epsilon), and δ (delta) for classes IgG, IgM, IgA, IgE, and IgD, respectively. A typical immunoglobulin (Ig) protein molecule has a sedimentation coefficient of about 7S and a molecular weight of about 150,000 daltons. The overall shape of an Ig molecule is modeled by the letter Y. All immunoglobulins have the same general structure. They are composed of four polypeptide chains, two that are heavy (H) and two light (L). The two heavy chains are joined together by two disulfide bridges known as the hinge region, which is approximately halfway along the chains. Closer to the amino terminal region, each heavy chain is joined by a disulfide bridge with a light chain. Each heavy chain has three constant regions, C_{H1}, C_{H2} and C_{H3}, which are the last two in the carboxy terminal region (before the hinge) and the first in the amino terminal region (immediately after the hinge). Each heavy chain also has a variable region (VH) in the amino terminal end. There are four such constant regions in classes IgM and IgE. Each light chain has only one constant region, CL, in the carboxy terminal end, and one variable region, VL, in the amino terminal end. Within an immunoglobulin class, all constant regions have homologous amino acid sequences. Heavy chains µ and ε lack hinge regions and contain an extra constant domain in their central region.

When IgG is digested enzymatically, different fragments maybe obtained, depending on the enzyme used. If papain is used, three fragments are obtained, the crystallizing fragment (Fc), and two antigen-binding fragments (Fab). If pepsin is used, one F(ab')₂ fragment is obtained, while the crystallizing fragment is digested. *(See* U.S. Patent Application Pub. No. 2002/0164327). This is due to the fact that papain cuts the heavy chains immediately after the hinge (toward the amino terminal region), while pepsin cuts them before the hinge (toward the carboxy terminal region). Fab and F(ab')₂ are fragments that conserve their capacity to specifically bind to the antigen that gave rise to them. F(ab')₂ will also still precipitate antigens, while the Fc antibody fraction normally acts as a marker signal for macrophages and in the activation of lymphocytes for the recognition and phagocytosis of the antigen-antibody complex.

The Fc fragment comprises the antigenic determinants of the antibody such that, when a patient is administered whole antibodies generated in an animal of another species, the patient generates an immune response against these antigenic determinants. This gives rise to varied adverse secondary responses that can even include anaphylactic shock. These problems are significantly reduced when the antibodies are digested with papain or pepsin, and only the resulting purified Fab or F(ab')₂ fragments are administered. This is due to the fact that these fragments lack antigenic determinants which would make the patient's organism recognize them as foreign.

The use of Fab or F(ab')₂ antibody fragments has another advantage with regard to distribution volume. Distribution volume is the volume of the body in which a determined drug is dissolved. This volume can refer to the circulating blood alone, as is the case with IgG, or may include a larger part of bodily water, as is the case with fragments. Due to the fact that Fab and F(ab')₂ have a greater corporal volume they can neutralize toxins lodged in various tissues, and are thus not restricted to travel through the blood. They are also able to cross the blood/brain barrier in both directions and may therefore be used to neutralize or eliminate neurotoxins.

The use of F(ab')₂ has a particular advantage over Fab in that they are retained far longer in the patient. This is due to the fact that F(ab')₂ fragments have double the molecular weight of Fabs, they conserve their capacity to precipitate the antigen in physiological conditions, and they maintain a size that allows them access to a distribution volume that is sufficient for treatment purposes.

One way a pharmaceutical composition of F(ab')₂ antibodies may be prepared is by immunizing groups of animals separately with the venom of snakes, scorpions, spiders or another venomous creature and subsequently bleeding the animals to obtain the antibodies produced in response to the immunization. The blood plasma of different animals immunized against the same polyvalent venom is mixed together, and the F(ab')₂ antibody fragments are collected after pepsin digestion and ammonium sulfate precipitation. The purified F(ab')₂ obtained for each of the venoms of the different genera is mixed in equal proportions before being formulated, dosified, and/or lyophilized in flasks.

F(ab')₂ antibody fragments specific to a particular cytokine may also be produced by hyperimmunization with a specific cytokine as described above, followed by pepsin and ammonium sulphate precipitation, but without mixing the purified anti-cytokine F(ab')₂ antibody fragment with fragments that are specific to other cytokines. For example, anti-TNF-α F(ab')₂ may be produced by immunizing an animal with TNF-α, and following the method described above. Purified F(ab')₂ specific to one cytokine may also be formulated, dosified and/or lyophilized in flasks for storage.

A "human in need of such treatment" may include a person who has come into contact with or been injected with a toxin such as the venom of a venomous creature. A human in need of treatment may also include a person suffering from other cytokine-mediated inflammatory diseases such as psoriasis vulgaris or rheumatoid arthritis. A "human in need of such treatment" may also include a person who has received a graft or organ transplant, who may experience a cytokine-mediated immune reaction such as acute transplant rejection.

The present invention is directed to reactions associated with other anti-venom treatments, possibly related to the presence of anticomplement activity (*i*.*e*. whole antibodies) or to lack of purity.

The antibody fragments are preferably free from albumin and of whole antibodies, as well as substantially free of pyrogens, and may be administered with an effective amount of an ophthalmologically acceptable carrier.

The anti-cytokine F(ab')₂ antibody fragments may be administered in a variety of delivery vehicles, which may include, but are not limited to: pourable liquids, ointments, emollients, creams, lotions, gels, wipes, solutions, sprays, powders, transdermal patches, bioadhesives, dressings, and pastes.

Another embodiment comprises a medicament for topical administration of a dermatologically acceptable liquid vehicle which comprises 0.1%-50% by weight anti-TNF-α F(ab')₂ antibody fragments. Another embodiment a medicament for topical administration of a dermatologically acceptable liquid vehicle which comprises 8%-35% by weight anti-TNF-α F(ab')₂ antibody fragments. Another embodiment comprises a medicament for topical administration of a dermatologically acceptable liquid vehicle which comprises 10%-25% by weight anti-TNF-α F(ab')₂ antibody fragments. In a preferred embodiment, the medicament comprises topical application of a dermatologically acceptable liquid vehicle which comprises 15%-20% by weight anti-TNF-α F(ab')₂ antibody fragments.

Another embodiment comprises a medicament for topical administration of a dermatologically acceptable gel vehicle which comprises 0.1%-50% by weight anti-TNF-α F(ab')₂ antibody fragments. Another embodiment comprises a medicament for topical administration ofa dermatologically acceptable gel vehicle which comprises 8%-35% by weight anti-TNF-α F(ab')₂ antibody fragments. Another embodiment comprises a medicament for topical administration of a dermatologically acceptable gel vehicle which comprises 10%-25% by weight anti-TNF-α F(ab')₂ antibody fragments. In a preferred embodiment, the medicament comprises topical application of a dermatologically acceptable gel vehicle which comprises 15%-20% by weight anti-TNF-α F(ab')₂ antibody fragments.

Another embodiment comprises a medicament for topical administration of a dermatologically acceptable semi-solid vehicle which comprises 0.1%-50% by weight anti-TNF-α F(ab')₂ antibody fragments. Another embodiment comprises a medicament for topical administration of a dermatologically acceptable semi-solid vehicle which comprises 8%-35% by weight anti-TNF-α F(ab')₂ antibody fragments. Another embodiment comprises a medicament for topical administration of a dermatologically acceptable semi-solid vehicle which comprises 10%-25% by weight anti-TNF-α F(ab')₂ antibody fragments. In a preferred embodiment, the medicament comprises topical application of a dermatologically acceptable semi-solid vehicle which comprises 15%-20% by weight anti-TNF-α F(ab')₂ antibody fragments.

In one embodiment, the medicament is applied within 24 hours of a transplant or graft. In another embodiment, the medicament is applied within 12 hours of a transplant or graft. In another embodiment, the medicament is applied within 2 hours of a transplant or graft. In a preferred embodiment, the medicament is applied or within a 30 minutes of a transplant or graft.

It is well known to those of ordinary skill in the art that drug-release from its vehicle is a function of concentration, solubility in the vehicle and the receptor site. Percutaneous absorption of the drug can also be enhanced by the use of occlusive techniques or by the use of penetration enhancers.

### EXAMPLES

In order to better illustrate the use of the present invention, the following specific examples are provided to assist the reader in the various aspects of practicing the present invention. As these specific examples are merely illustrative, nothing in the following descriptions should be construed as limiting the invention in any way. All percentages of the components comprising the invention are herein referred to their weight in each composition as a whole, unless otherwise noted.

### EXAMPLE 1

Topical pharmaceutical base with F(ab')₂ fragments. The composition administered in this example takes the form of a topical cream. The composition comprises: (a) about 1% to 40% by weight urea; (b) about 0.0 1 % to about 1% by weight of an astringent such as calcium acetate, ammonium sulfate or a mixture thereof; (c) about 0.01% to about 1% by weight of an anesthetic agent and dermatologically acceptable excipients; and (d) 0.1% to 50% by weight of anti-IFN-γ anti-cytokine F(ab')₂ antibody fragments.

### EXAMPLE 2

Administration with enhanced penetration and reduced irritation. The penetration enhancing system of the composition applied in the method of this example effectively enhances transdermal delivery of anti-cytokine F(ab')₂ fragments, while reducing skin irritation. The composition comprises: (a) anti-cytokine F(ab')₂ antibody fragments (the active agent) having 0.1- 50 weight percent of the total composition; (b) a penetration-enhancing system consisting essentially of (i) a membrane fluidizer comprising oleic acid; (ii) a C₁-C₄ alcohol; and (iii) a glycol having a pH between 4 and 8. The composition administered in the method of the present example may also be prepared with a gelling agent for increased viscosity.

### EXAMPLE 3

The method of the present example permits the efficient and continuous release of anti-cytokine F(ab')₂ antibody fragments onto the surface of the skin and enhances penetration into the epidermis and underlying dermal and subcutaneous tissues. The composition comprises: (a) about 1% laureth-4 (or another surface tension reducing agent); (b) about 2% propylene glycol (or an alternative skin hydrating agent); (c) about 0.5% dimethylsorbide (or alternative hydrophilic-lipophilic coupling agent; (d) 0.1% to 50% anti-cytokine F(ab')₂ antibody fragments; and (e) a pharmaceutically acceptable diluent comprising a mixture of water and ethanol.

### EXAMPLE 4

Prevention of tissue transplant rejection. This example describes a method of preventing the rejection of transplanted tissue caused by proinflammatory cytokines that are involved in the alloimmune response. TNF-α is one such cytokine. Thus, a composition as described in Example 1, 2 or 3 comprising around 40% by weight anti-TNF-α F(ab')₂ antibody fragments is topically applied to a graft recipient 3 times a day for 8 weeks beginning 24 hours after transplantation. The anti-TNF-α F(ab')₂ antibody fragments are absorbed into the tissue surrounding the graft as well as the graft tissue itself to neutralize cytokines such as TNF-α, and promote healing with little or no graft rejection.

### EXAMPLE 5

Prevention of corneal transplant rejection. This example describes a method of preventing the rejection of transplanted tissue, specifically corneal tissue, caused by proinflammatory cytokines that are involved in the alloimmune response. The composition as described in Example 1, 2 or 3 comprising 20-30mg/ml anti-TNF-α F(ab')₂ antibody fragments in an opthalmic suspension is applied directly to the eye. Within 24 hours of the corneal transplant surgery, the first dose of composition is applied. With a buffered saline solution vehicle which may also include antimicrobial and stabilizing agents, the first step is to gently pull the lower eyelid down and drop the composition in the lower lid. The lower lid is released and blinking should be avoided for at least 30 seconds. This process is repeated every 2 to 3 hours for up to 8 weeks to prevent corneal graft rejection.

Alternatively, the F(ab')₂ anti-body fragments may be delivered through an ophthalmic suspension or ointment. The anti-TNF-α F(ab')₂ antibody fragments are put into suspension under sterile conditions and are applied within the first 24 hours of the corneal transplant surgery. Such a suspension is typically stored in a collapsible tube, which may be used to administer the ointment directly to the eye. In order to apply the ointment, gently pull the lower eyelid down. While looking up (if possible), squeeze a small amount of ointment (about 1/2 to 1/4 inch) inside lower lid. Close the eye and gently and roll the eyeball in all directions while the eye is closed. Temporary blurring may occur. The closed eyelid may be rubbed very gently by a finger to distribute the drug throughout the fornix. This process may be repeated every 10 to 12 hours for up to 8 weeks to prevent corneal graft rejection. Ophthalmic ointment administration should be limited to bedtime instillation as ointments will interfere with vision.

## Claims

1. Use of an anti-TNF-α F(ab')₂ neutralizing antibody fragment in the preparation of a medicament for topical administration for treating an TNF-α-mediated immune reaction which is due to a rejection of a corneal transplant wherein the medicament is prepared for direct application to the eye.

2. The use of claim 1, wherein said anti-TNF-α F(ab')₂ antibody fragment is free of albumin, whole antibodies, and/or pyrogens.

3. The use of claim 1, wherein the medicament comprises an ophthalmologically acceptable carrier.

4. Anti-TNF-α F(ab')₂ neutralizing antibody fragment for use in treating an TNF-α-mediated immune reaction which is due to a rejection of a corneal transplant, wherein the antibody fragment is comprised by a medicament for topical administration and wherein the medicament is prepared for direct application to the eye.

5. Anti-TNF-α F(ab')₂ neutralizing antibody fragment of claim 4 for use in treating an TNF-α-mediated immune reaction which is due to a rejection of a corneal transplant, wherein said anti-TNF-α F(ab')₂ antibody fragment is free of albumin, whole antibodies, and/or pyrogens

6. Anti-TNF-α F(ab')₂ neutralizing antibody fragment of claim 4 for use in treating an TNF-α-mediated immune reaction which is due to a rejection of a corneal transplant, wherein the medicament comprises an ophthalmologically acceptable carrier.

## Patentansprüche

1. Verwendung eines neutralisierenden anti-TNF-α F(ab')₂ Antikörperfragmentes zur Herstellung eines topisch anzuwendenden Arzneimittels zur Behandlung einer TNF-αvermittelten Immunreaktion, die auf eine Abstoßung eines Hornhauttransplantates zurückzuführen ist, wobei das Arzneimittel für die direkte Applikation am Auge zubereitet ist.

2. Verwendung gemäß Anspruch 1, wobei das anti-TNF-α F(ab')₂ Antikörperfragment frei von Albumin, ganzen Antikörpern und/oder Pyrogenen ist.

3. Verwendung gemäß Anspruch 1, wobei das Arzneimittel einen ophthalmologisch verträglichen Träger umfasst.

4. Neutralisierendes anti-TNF-α F(ab')₂ Antikörperfragment zur Verwendung bei der Behandlung einer TNF-α-vermittelten Immunreaktion, die auf eine Abstoßung eine Hornhauttransplantates zurückzuführen ist, wobei das Antikörperfragment in einem topisch anzuwendenden Arzneimittel enthalten ist und wobei das Arzneimittel für die direkte Applikation am Auge zubereitet ist.

5. Neutralisierendes anti-TNF-α F(ab')₂ Antikörperfragment gemäß Anspruch 4 zur Verwendung bei der Behandlung einer TNF-α-vermittelten Immunreaktion, welche die Folge einer Abstoßung eines Hornhauttransplantates ist, wobei das anti-TNF-α F(ab')₂ Antikörperfragment frei von Albumin, ganzen Antikörpern und/oder Pyrogenen ist.

6. Neutralisierendes anti-TNF-α F(ab')₂ Antikörperfragment gemäß Anspruch 4 zur Verwendung bei der Behandlung einer TNF-α-vermittelten Immunreaktion, welche die Folge einer Abstoßung eines Hornhauttransplantates ist, wobei das Medikament einen ophthalmologisch verträglichen Träger umfasst.

## Revendications

1. Utilisation d'un fragment d'anticorps neutralisant F(ab')₂ anti-TNF-α dans la préparation d'un médicament pour l'administration topique pour traiter une réaction immunitaire médiée par le TNF-α qui est due à un rejet d'un transplant cornéen, dans laquelle le médicament est préparé pour une application directe sur l'oeil.

2. Utilisation selon la revendication 1, dans laquelle ledit fragment d'anticorps F(ab')₂ anti-TNF-α est exempt d'albumine, d'anticorps entiers et/ou de pyrogènes.

3. Utilisation selon la revendication 1, dans laquelle le médicament comprend un support ophtalmologiquement acceptable.

4. Fragment d'anticorps neutralisant F(ab')₂ anti-TNF-α pour une utilisation dans le traitement d'une réaction immunitaire médiée par le TNF-α qui est due à un rejet d'un transplant cornéen, lequel fragment d'anticorps est compris dans un médicament pour une administration topique et lequel médicament est préparé pour une application directe sur l'oeil.

5. Fragment d'anticorps neutralisant F(ab')₂ anti-TNF-α selon la revendication 4, pour une utilisation dans le traitement d'une réaction immunitaire médiée par le TNF-α qui est due à un rejet d'un transplant cornéen, lequel fragment d'anticorps F(ab')₂ anti-TNF-α étant exempt d'albumine, d'anticorps entiers et/ou de pyrogènes.

6. Fragment d'anticorps neutralisant F(ab')₂ anti-TNF-α selon la revendication 4, pour une utilisation dans le traitement d'une réaction immunitaire médiée par le TNF-α qui est due à un rejet d'un transplant cornéen, dans lequel le médicament comprend un support ophtalmologiquement acceptable.
